# EUROPEAN PATENT SPECIFICATION

(11) **EP 3 047 827 B1**
(45) Date of publication and mention of the grant of the patent: **28.03.2018**
(21) Application number: 14820118.9
(22) Date of filing: 04.04.2014
(51) Int. Cl.: A61F 13/15

(54) **PACKAGING BODY FOR URINE ABSORPTION PAD FOR MEN**
VERPACKUNGSKÖRPER FÜR URINABSORPTIONSKISSEN FÜR MÄNNER
EMBALLAGE POUR GARNITURE DE COLLECTE D'URINE POUR HOMME

(30) Priority: 20.09.2013 JP 2013196228
(43) Date of publication of application: 27.07.2016
(73) Proprietor: Unicharm Corporation, Ehime 799-0111 (JP)
(72) Inventor: FUJIMOTO, Kazuya, Kanonji-shi Kagawa 769-1602 (JP); NAKAJIMA, Kaiyo, Kanonji-shi Kagawa 769-1602 (JP)
(74) Representative: Peter, Julian
(86) International application number: PCT/JP2014/059930
(87) International publication number: WO 2015/001825

(56) References cited:
- WO-A1-99/60965
- WO-A1-99/60965
- WO-A1-2010/071517
- WO-A1-2011/162657
- WO-A1-2013/114839
- JP-A- H09 506 280
- JP-A- H10 127 686
- JP-A- 2003 088 548
- JP-A- 2008 154 606
- JP-A- 2008 245 841
- JP-A- 2009 006 019
- JP-A- 2010 172 650
- JP-A- 2011 120 711
- JP-A- 2013 172 846
- JP-B1- 5 433 815
- US-B1- 6 565 548
- US-B1- 8 202 264

## Description

### {Technical Field}

The present invention relates to individually packaged male urine absorption pads, each including a male urine absorption pad and an envelope individually packaging the same.

### {Background}

Individually packaged bodily-fluid absorption pads, particularly male urine absorption pads, are conventionally known. For example, an individually packaged male urine absorption pad disclosed in Patent Literature 1 includes an absorption pad having a longitudinal direction, a width direction, a skin-facing surface and a non-skin-facing surface opposed thereto. The absorption pad is shaped in such a manner that one of both end portions in the longitudinal direction is wider than the other, and an envelope individually packaging the absorption pad in a folded-back state.
Further prior art in this technical field is disclosed in WO 99/60965 A1 and WO 2013/114839 A1.

### {Citation List}

### {Patent Literature}

{PTL 1}: JP H10-127686 A

### {Summary}

### {Technical Problem}

For the individually packaged absorption pad disclosed in Patent Literature 1, the absorption pad is folded back in relatively wide both lateral regions thereof along fold lines extending in the longitudinal direction, then folded back in both end regions along fold lines extending in the lateral direction and thereafter the absorption pad is individually packaged in the envelope for this purpose. Such individually packaged pad is better in the portability since the pad is packaged in a compactly folded back state. However, the absorption pad having been folded back by the above-mentioned process inevitably has a folding propensity in the longitudinal direction remarkably left thereon. Consequently, the absorption pad is likely flexed along the fold lines in the longitudinal direction under an external force in the lateral direction after the pad has been put on the wearer's body.

In general, for the male urine absorption pad worn on the male sexual organ, it has not been assumed until recently that such pad may be carried by its user whenever leaving home as has already been assumed for female sanitary articles such as sanitary napkins and the breast pads. For this reason, the individually packaged male urine absorption pads have not been distributed in the market until quite recently. However, because of the aging population, the needs for the individually packaged male urine absorption pads are increasing from the viewpoint of portability whenever leaving home, convenience and good hygiene.

Considering that the male urine absorption pad has a contour larger than the female articles such as the breast pads and the sanitary napkins, it is necessary to fold back the pad more than once prior to insertion of the pad into the packaging envelope so that the individually packaged urine absorption pad may be sufficiently compact to assure a high portability. For example, assuming that the packaging method disclosed in Patent Literature 1 is used to obtain the individually packaged male urine absorption pad, the folding propensity in the longitudinal direction is remarkably left on the pad and the pad may be readily flexed and make it difficult for the entire male urine absorption pad to be convexly curved along the wearer's body. Consequently, not only a fit and a wear comfort may be deteriorated but also body exudates may leak through a gap left between the wearer's body and the pad.

An object of the present invention is to improve the prior art providing an individually packaged male urine absorption pad which is deformable along the wearer's body after the pad has been taken out from the envelope and put on the wearer's body, and excellent in portability.

### {Solution to Problem}

The present invention to solve the problem set forth above is defined by a package according to claim 1.

### {Advantageous Effects of Invention}

According to one or more embodiments of the present invention, the male urine absorption pad is individually packaged, and whereby not only a portability but also hygienic aspect is improved. In addition, after the pad is folded back along a pair of first fold lines and thereby first, second and third regions arranged in a longitudinal direction are folded back on one another, the male urine absorption pad is packaged. In this manner, both the lateral regions of the pad main body are folded back after the central region in the process of packaging. In consequence, the folded regions along the first fold lines are substantially free from a folding propensity and, in the pad put on the wearer's body, it is assured that the pad main body is smoothly deformed along the wearer's body shape.

### {Brief Description of Drawings}

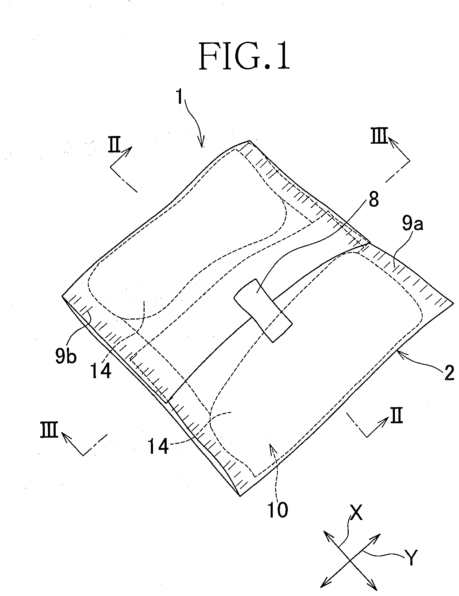

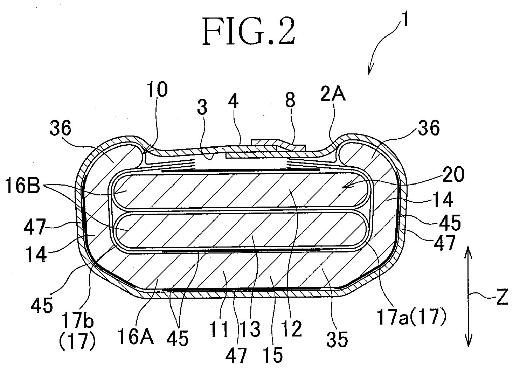

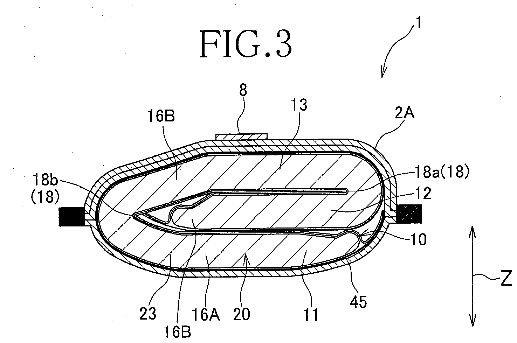

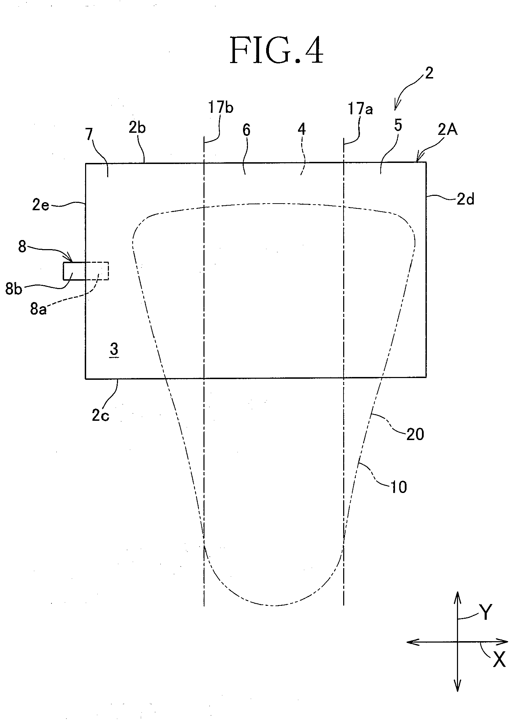

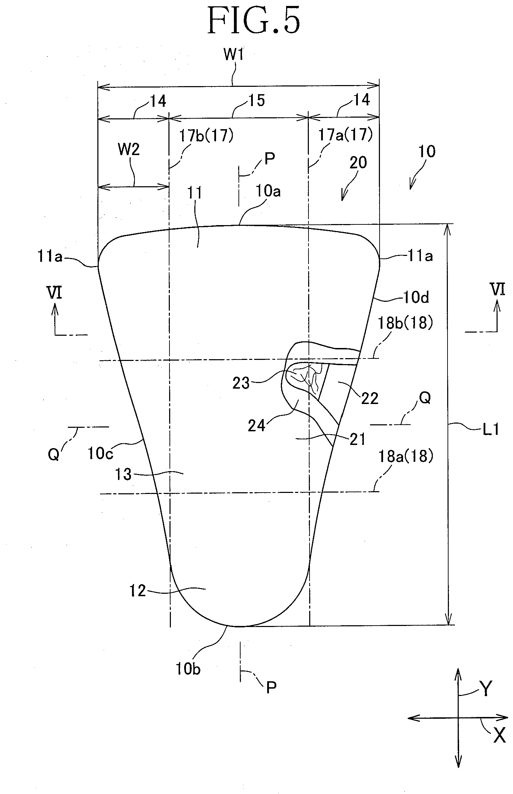

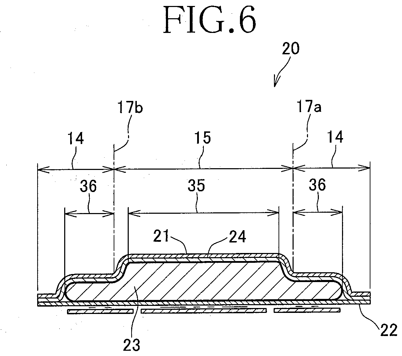

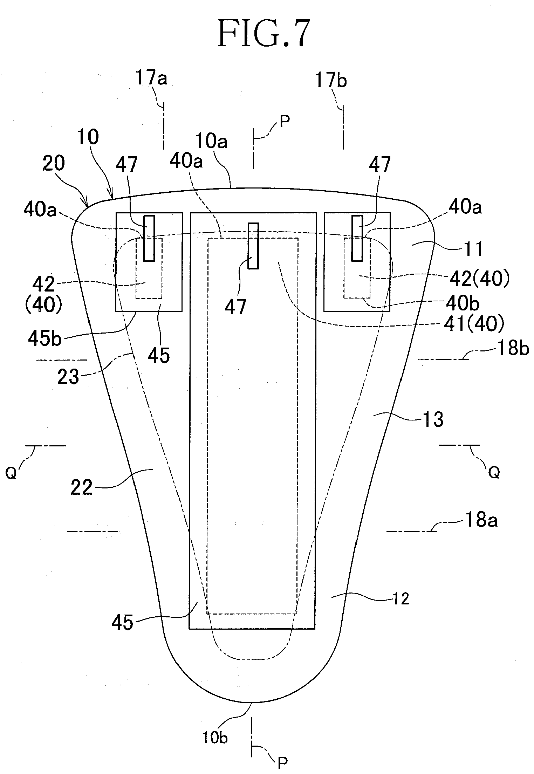

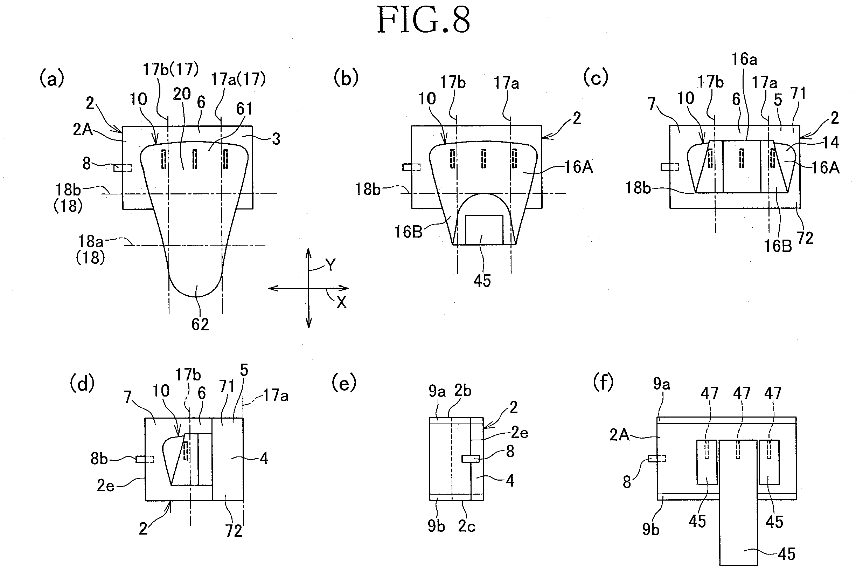

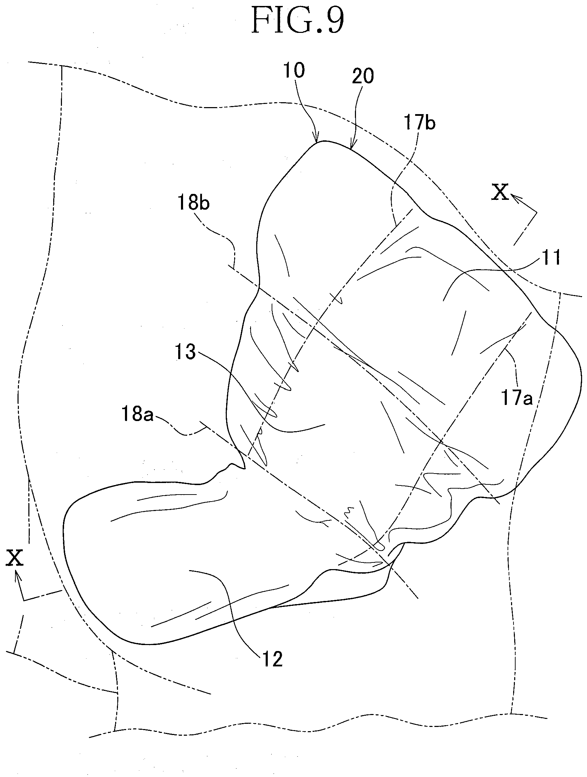

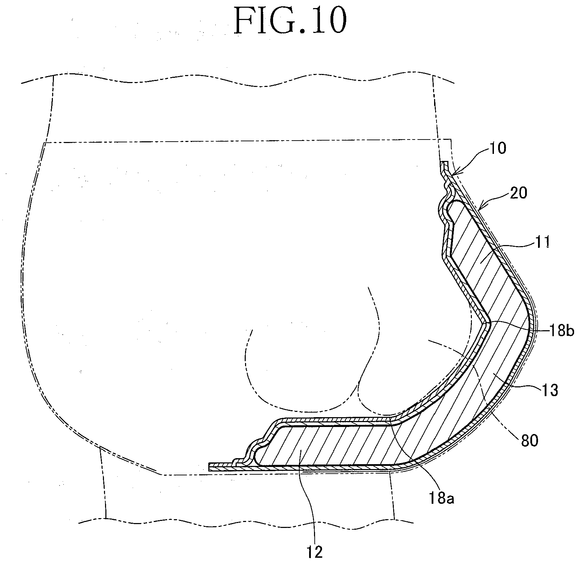

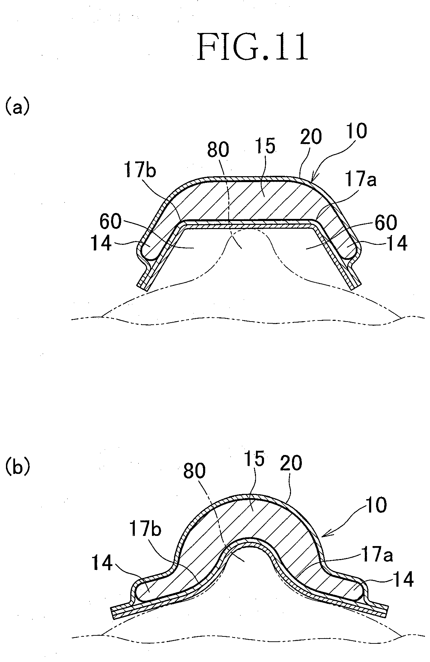

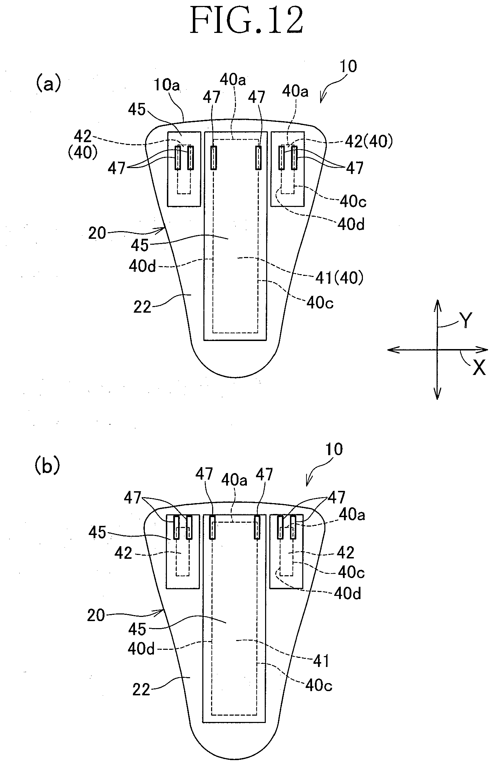

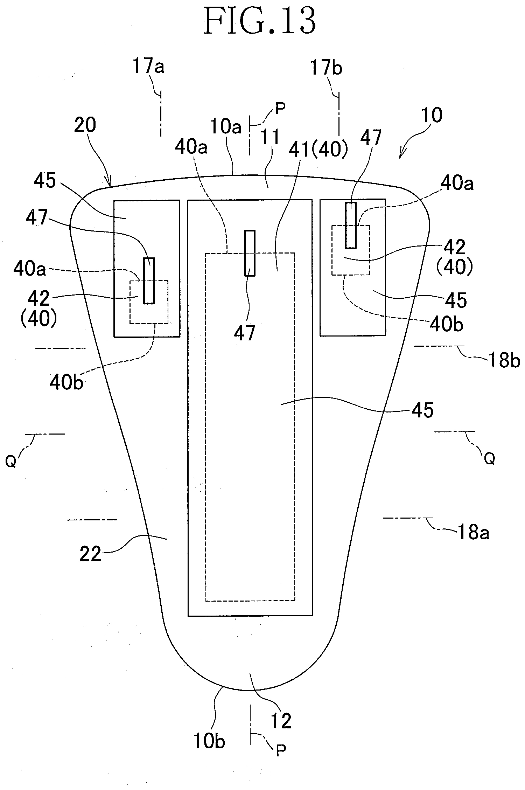

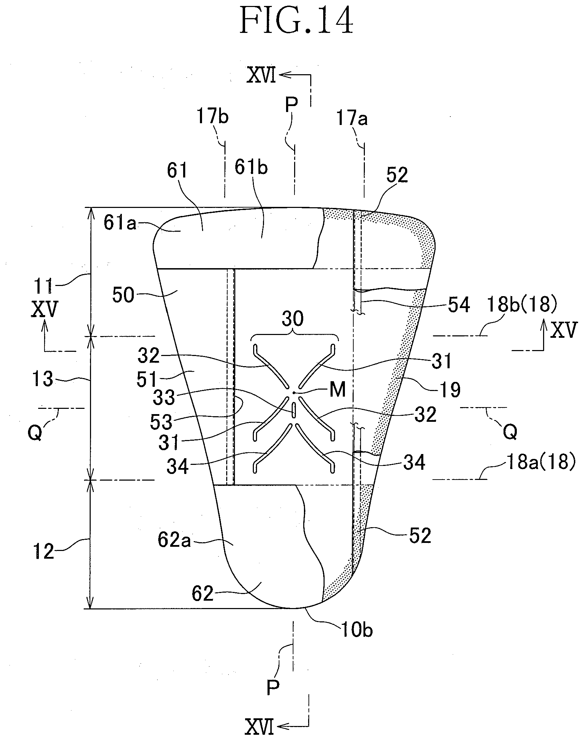

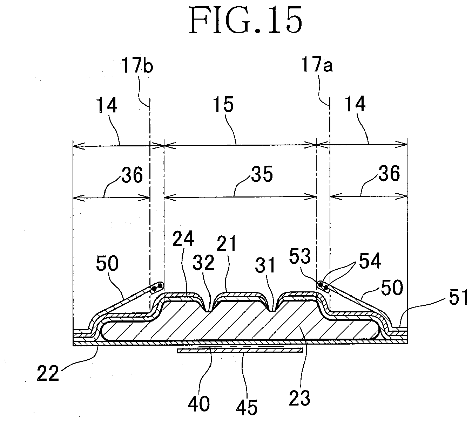

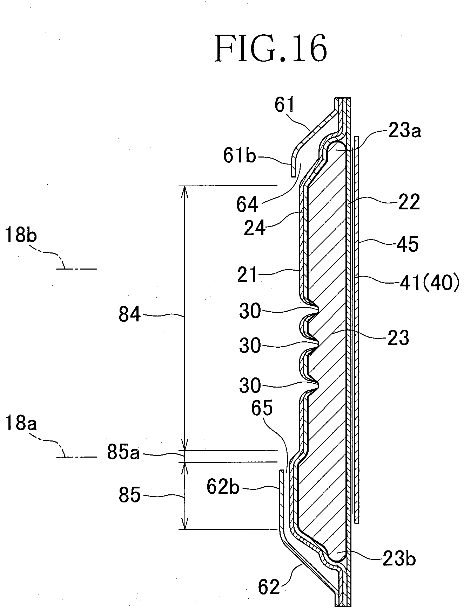

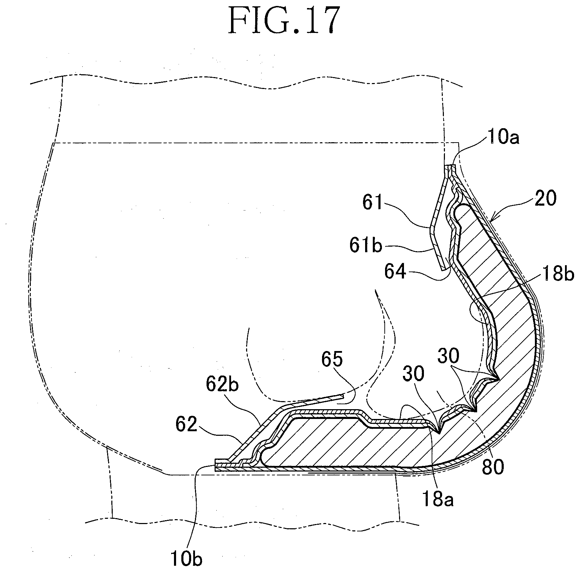

The drawings illustrate specific embodiments of the present invention including optional and preferred embodiments as well as essential features of the invention.
{Fig. 1} Perspective view exemplarily illustrating an individually packaged male urine absorption pad according to a first embodiment of the present invention.
{Fig. 2} A schematic sectional view taken along line II-II in Fig. 1.
{Fig. 3} A schematic sectional view taken along line III-III in Fig. 1.
{Fig. 4} A Plan view of flatly developed envelope used for individually packaging.
{Fig. 5} A partially cutaway flatly developed plan view of a male urine absorption pad.
{Fig. 6} A schematic sectional view taken along line VI-VI in Fig. 5.
{Fig. 7} A bottom plan view of the male urine absorption pad.
{Fig. 8} (a) through (f): Diagrams illustrating a packaging process and a taking-out process for the male urine absorption pad.
{Fig. 9} A perspective view exemplarily illustrating the male urine absorption pad put on the wearer's body.
{Fig. 10} A schematic sectional view taken along line X-X in Fig. 9.
{Fig. 11} (a): A sectional view exemplarily illustrating the male urine absorption pad packaged in folded back state according to the conventional method and put on the wearer's body and (b): a sectional view exemplarily illustrating the male urine absorption pad according to the present invention put on the wearer's body.
{Fig. 12} A view similar to Fig. 7, illustrating an alternative of the individually packaged male urine absorption pad.
{Fig. 13} A bottom plan view of the male urine absorption pad according to a second embodiment.
{Fig. 14} A flatly developed planar view illustrating the male urine absorption pad according to a third embodiment.
{Fig. 15} A schematic sectional view taken along line XV-XV in Fig. 14.
{Fig. 16} A schematic sectional view taken along line XVI-XVI in Fig. 4.
{Fig. 17} A diagram illustrating a state of the male urine absorption pad illustrated in Fig. 14 put on the wearer's body.

### {Description of Embodiments}

The embodiments described below is directed to the individually packaged male urine absorption pad illustrated in Figs. 1 through 17 including optional and preferred arrangements as well as essential arrangements of the invention.

### <First Embodiment>

Fig. 1 exemplarily illustrates a package 1 of male urine absorption pad. Referring to Figs. 1 through 3, the package 1 includes a male urine absorption pad 10 having a longitudinal direction X, a width direction Y being orthogonal thereto and a pad main body 20 defining a general shape of the package 1, and an individually packaging envelope 2 individually packaging the male urine absorption pad 10. The package 1 has a pair of first fold lines 17a, 17b extending in the width direction Y and a pair of second fold lines 18a, 18b extending in the longitudinal direction X. The first fold lines 17a, 17b are the lines along which the individually packaging envelope 2 is folded together with the male urine absorption pad 10 and the second fold lines 18a, 18b are the lines along which the male urine absorption pad 10 only is folded.

Referring to Fig. 4, the individually packaging envelope 2 is formed of a packaging sheet 2A shaped into a rectangle relatively longer in the longitudinal direction X and contoured by first and second side edges 2b, 2c extending in the longitudinal direction X and first and second end edges 2d, 2e extending in the width direction Y. The individually packaging envelope 2 is divided into a first region 5 defined between the first fold line 17a and the first end edge 2d, a second region 6 defined between the first fold line 17a and the first fold line 17b and a third region 7 defined between the first fold line 17b and the second end edge 2e. The packaging sheet 2A has a first surface 3 on which the male urine absorption pad 10 is located and a second surface 4 opposite thereto. A finger-grip tab 8 used to unwrap the package 1 is fixed to the second surface 4 of the packaging sheet 2A in the vicinity of the second end edge 2e in a manner such that the finger-grip tab 8 strides across the second end edge 2e. The finger-grip tab 8 has a fixed portion 8a fixed to the second surface 4 and a free portion 8b extending outward from the fixed portion 8a.

The packaging sheet 2A may be formed of plastic films or preferably fibrous nonwoven fabrics made of thermoplastic synthetic fibers, particularly, spun bond/melt blown/spun bond (SMS) laminated fibrous nonwoven fabrics and each of these fibrous nonwoven fabrics preferably has a mass per unit area ranging about 10 to about 30 g/m². It is also possible to use a laminate of these plastic films and fibrous nonwoven fabrics.

Referring to Fig. 5, the pad main body 20 has a longitudinal center line P, a lateral center line Q, a skin-facing surface, a non-skin-facing surface opposite thereto, curved first and second end edges 10a, 10b and both side edges 10c, 10d extending between these first and second end edges, 10a, 10b, respectively. The male urine absorption pad 10 has an external shape such that a length dimension in a direction of the lateral center line Q (corresponding to the longitudinal direction X of the packaging sheet 2A) of the first end edge 10a is larger than that of the second end edge 10b so as to be tapered from the first end edge 10a toward the second end edge 10b. The male urine absorption pad 10 shaped in this manner has a pair of second fold lines 18 extending in the lateral center line Q and a pair of first fold lines 17 extending across the second fold lines 18 in the direction of the longitudinal center line P.

In the direction of the longitudinal center line P, the pad main body 20 is divided into a first end region 11 extending from one of the second fold lines 18b toward the first end edge 10a, a second end region 12 extending from the other second fold line 18a toward the second end region 10b and an intermediate region 13 defined between the first and second end regions 11, 12. In the direction of the lateral center line Q, the male urine absorption pad 10 is divided into both lateral regions 14 defined between the pair of the first fold lines 17a, 17b and both the side edges 10c, 10d facing the respective first fold lines 17a, 17b, and a central region 15 defined between both the lateral regions 14. In the first end region 11, a dimension W2 in the direction of the lateral center line Q from the end edge 11a to the first fold line 17 is preferably in a range of 15 to 35% of a dimension W1 in the direction of the lateral center line Q of the male urine absorption pad 10 as a whole.

Although the pad main body 20 is folded in three along the pair of second fold lines 18a, 18b according to the present embodiment, it is not needed to arrange a pair of the second fold lines extending in the direction of the lateral center line Q as long as the number of the fold lines is two or more. For example, it is also possible to arrange three second fold lines and to package the pad in a state folded in four.

A dimension L1 in the longitudinal center line P of the pad main body 20 (the male urine absorption pad 10 also) is preferably in a range of about 160 mm to about 350 mm. If the dimension L1 is less than 160 mm, the penis of the average adult may not be properly wrapped up and, if the dimension L1 exceeds 350 mm, the pad main body may extend to the vicinity of the anus and provide a discomfort. The dimension W1 in the direction of the lateral center line Q of the pad main body 20 (the male urine absorption pad 10 also) is preferably in a range of 120 mm to 300 mm. A dimension from the first end edge 10a to the second fold line 18b on the longitudinal center line P and a dimension from the second end edge 10b to the second fold line 18a are preferably in a range of 15 to 40% of the dimension L of the pad main body 20 as a whole in the direction of the longitudinal center line P, respectively. If the dimension from the second end edge 10b to the second fold line 18a is less than 15% of the dimension L1, a space defined by the pad in the vicinity of the wearer's inguinal region may be too limited to wrap up the scrotum. If the above-mentioned dimension exceeds 40% of the dimension L1, the dimension in the direction of the longitudinal center line P of the intermediate region 13 may become too short and, in consequence, the space to receive the penis in the intermediate region 13 may be too limited to wrap up the penis stably.

The pad main body 20 includes a topsheet 21 on the side of the skin-facing surface, a backsheet 22 on the side of the non-skin-facing surface and a liquid-absorbent core 23 interposed between the topsheet 21 and the backsheet 22. Optionally, a cushion sheet 24 is interposed between the topsheet 21 and the liquid-absorbent core 23. In the pad main body 20, the topsheet 21, the cushion sheet 24 and the backsheet 22 extend outward beyond an outer peripheral edge of the liquid-absorbent core 23 and bonded to each other along the outer peripheral edge of the pad main body 20 by known bonding means such as hot melt adhesives and heat-bond techniques. The cushion sheet 24 is preferably formed of sheet materials having properties to prevent the body exudates from flowing back (causing rewet).

The topsheet 21 and the cushion sheet 24 may be formed of, for example, liquid-permeable fibrous nonwoven fabrics. The backsheet 22 may be formed of hardly-liquid-permeable or liquid-impermeable fibrous nonwoven fabrics, air-permeable plastic films or a laminate sheet of these fibrous nonwoven fabrics and plastic films. It is possible to arrange coloring element in the central region 15 of the cushion sheet 24 so that such coloring element may be seen through the topsheet 21. The coloring element arranged in the central region 15 of the cushion sheet 24 makes it easy for the wearer to position his penis in contact with the central region 15 when the wearer tries to put his penis in contact with the interior surface of the pad main body 20.

The liquid-absorbent core 23 is molded in substantially the same shape as that of the pad main body 20 and includes absorbent material formed of a mixture of wood fluff pulps, superabsorbent polymer particles (SAP) and, optionally, thermoplastic fibers and a liquid-diffusive sheet such as tissue paper used to wrap up the core materials.

Referring to Fig. 6, the liquid-absorbent core 23 has a central region 35 as viewed in the direction of the lateral center line Q and both lateral regions 36 defined on both sides of the central region 35. A thickness of the liquid-absorbent core 23 in the central region 35 is larger than that in both the lateral regions 36 and, in the vicinity of boundaries between the central region 35 and the lateral regions 36, the thickness of the liquid-absorbent core 23 is changed so as to be gradually reduced from the central region 35 to both the lateral regions 36. The central region 35 of the liquid-absorbent core 23 overlaps with the central region 15 of the pad main body 20 in a thickness direction Z. The first fold lines 17 are located on the respective outer sides of the central region 35 as viewed in the lateral center line Q and, for the present embodiment, the first fold lines 17 are located to overlap with the respective lateral regions 36 and, in portions of the core 23 defined outside the respective first fold lines 17 as viewed in the direction of the lateral center line Q, i.e., in both the lateral regions 14 of the pad main body 20, thickness and stiffness of the pad main body 20 lower than in the central region 15. In consequence, both the entire lateral regions 14 are freely deformable and gently flexed in the folded state of the pad. In this manner, the folding propensity may be left after the package has been unwrapped is restricted furthermore reliably.

A mass per unit area in the central region 35 of the liquid-absorbent core 23 is preferably in a range of about 150 g/m² to about 600 g/m² and a mass per unit area in both the lateral regions 36 is preferably in a range of 100 g/m² to 250 g/m². If the mass per unit area in the central region 35 is less than 150 g/m², the stiffness may be excessively reduced to maintain the shape and, if the mass per unit area in this region is more than 600 g/m², the pad may become too bulky and the wearer may experience a discomfort when the pad is put on the wearer's body. If a mass per unit area of the lateral region 36 exceeds 250 g/m², the stiffness may become excessively high to fold the male urine absorption pad 10 along the fold lines 17 and to package the pad 10. The mass per unit area of both the lateral regions 36 of the liquid-absorbent core 23 may be kept to be less than the mass per unit area of the central region 35 in this manner to make both the lateral regions 14 of the pad main body 20 freely deformable, thereby providing a good fit to the wearer's body.

The shape of the liquid-absorbent core 23 in which a thickness in the central region 35 and a thickness in the respective lateral regions 36 are relatively different from each other may be obtained, for example, with use of methods as follows: (1) the core material is deposited into a mold cavity having a shape similar to that of the liquid-absorbent core until the cavity is filled with the core material; (2) the mold having the shape similar to that of the liquid-absorbent core 23 is pressed against the core material having an appropriate thickness until the latter is compressed to the desired thickness. According to the method (1), it is possible to even up densities (g/cm³) of the central region 35 and the respective lateral regions 36, at least substantially. According to the method (2), it is possible to set the density (g/cm³) of the respective lateral regions 36 to a level higher than that of the central region 35 since the core material in the lateral regions 36 are compressed by the mold more strongly than the central region 36.

Referring to Fig. 7, a plurality of linear attachment regions 40 formed of pressure-sensitive adhesive and extending in the direction of the longitudinal center line P at given intervals in the direction of the lateral center line Q are arranged on the underside surface of the pad main body 20, i.e., the exterior surface of the backsheet 22 so that the pad main body 20 may be attached to the wearer's garment such as the underwear. The attachment regions 40 include a central attachment region 41 extending across the lateral center line Q from the first end region 11 to the second end region 12 and a pair of lateral attachment regions 42 arranged in the first end region 11, on both sides of the central attachment region 41. The attachment regions 40 are respectively covered with separators 45 formed of plastic sheets or fibrous nonwoven fabrics. The central attachment region 41 and the lateral attachment regions 42 respectively have end edges 40a on the side of the first end edge 10a of the pad main body 20 aligned with each other in the direction of the lateral center line Q.

The central attachment region 41 in the direction of the lateral center line Q of the pad main body 20 has a dimension in the direction of the lateral center line Q (i.e., a width dimension) which is 40 mm or more, preferably in a range of about 40 to about 125 mm. In the pad put on the wearer's body, the central attachment region 41 is attached to the garment such as the undergarment and defines a planar bottom of the cup-shaped pad main body 20. The above-mentioned width dimension may be set to 40 mm or more to ensure the relatively large planar bottom and thereby makes it possible to get a cup-like shape having a sufficient space to wrap up the penis. In order that the used pad main body 20 is easily peeled off from the garment with any one of the first or second end regions 11, 12 gripped in the fingers, the central attachment region 41 is located preferably at a distance in a region of 15 to 90 mm, more preferably in a range of 20 to 55 mm from the first and second end edges 10a, 10b of the pad main body 20.

Each of the lateral attachment regions 42 preferably has a dimension in the direction of the lateral center line Q (i.e., a width dimension) in a range of 5 to 62.5 mm, located at a distance in a range of 15 to 85 mm from the first end edge 10a and at a distance in a range of 10 to 85 mm in the direction of the lateral center line Q from the central attachment region 41. Preferably, end edges 40b of the respective lateral attachment regions 42 on the side of the second end edge 10b extend not to the second fold line 18b. Respective end edges 45b of separators 45 covering the respective lateral attachment regions 42 on the side of the second end region 12 preferably extend not beyond the second fold line 18b. The lateral attachment regions 42 and the separators 45 covering them may be arranged within the first end region 11 in this manner to assure that the separators 45 associated with the respective lateral attachment regions 42 are prevented from being rolled up even when the male urine absorption pad 10 is folded along the second fold line 18b.

A rear surface of the separator 45, i.e., the surface not facing the attachment region 40 is partially coated with adhesives so as to form adhesive portion 47 and each of the separators 45 is bonded to the packaging sheet 2A by such adhesive portion 47. The adhesive portions 47 are formed so as to overlap partially with the associated attachment regions 40 in the thickness direction Z of the male urine absorption pad 10 (the direction orthogonal to the X-direction and Y-direction). According to the present embodiment, the adhesive portions 47 are located on the side of the end edge 40a of the central attachment region 41 and on the side of the end edge 40a of the respective lateral attachment regions 42 so as to intersect with the end edge 40a and to extend outward in the longitudinal center line P beyond the end edge 40a. A peel resistance of the individual packaging envelope 2 to the adhesive portions 47 is higher than a peel resistance of the separators 45 to the attachment regions 40.

A method of packaging the male urine absorption pad 10 arranged as has been described above into the individually packaging envelope 2 is described below.

First, as illustrated in Figs. 4 and 8(a), the pad main body 20 is placed on the packaging sheet 2A in such a manner that the first end region 11 of the pad main body 20 may face the second region 6 in the first surface 3 of the packaging sheet 2A. On the first surface 3 of the packaging sheet 2A, the adhesive portions 47 are arranged at given intervals in the longitudinal direction X and exterior surfaces of the respective separators 45 are attached to the first surface 3 of the packaging sheet 2A. In this way, the male urine absorption pad 10 is fixed to the first surface 3 of the packaging sheet 2A. In this regard, it is not needed that the first surface 3 of the packaging sheet A is preliminarily provided with the adhesive portions 47 and it is also possible to provide the male urine absorption pad 10 on the exterior surface of the respective separators 45 with such adhesive portions 47.

Referring to Figs. 8(b) and 8(c), after the first end region 11 (a first layer 16A) of the pad main body 20 is fixed to the first surface 3 of the packaging sheet 2A through the intermediary of the adhesive portions 47, the second end region 12 is folded back along the second fold line 18a onto the skin-facing surface of the intermediate region 13 so as to form a layered section. Then the layered section (a second layer 16B) including the second end region 12 and the intermediate region 13 is folded back along the second fold line 18b onto the first end region 11 fixed to the first surface 3 of the packaging sheet 2A. By folding the pad main body 20 in three in the width direction Y of the packaging sheet 2A, the form of the pad main body 20 becomes compact, specifically, smaller than a width dimension of the individually packaging envelope 2. Though a crease formed along the second fold line 18a of the pad main body 20 overlaps with the first end edge 10a according to the present embodiment, this crease may be located on the outer side of the first end edge 10a or on the inner side thereof within a scope of the present invention.

Referring to Figs. 8 (c) through Fig. 8 (e), in the individually packaging envelope 2 having the first surface 3 on which the pad main body 20 folded in three is placed, the first region 5 is folded along the first fold line 17a onto the first surface 3 of the second region 6 so as to form a layered section. Then, the third region 7 is folded along the first fold line 17b onto the layered section of the first and second regions 5, 6. Assuming that a dimension in the width direction (direction X) of the pad main body 20 is uniform over the range from the first end edge 10a to the second end edge 10b, a thickness of both the lateral regions 14 after the second layer 16B is layered on the first layer 16A might become too thick to fold both the lateral regions 14 neatly along the first fold lines 17a, 17b. According to the present embodiment, the width dimension of the pad main body 20 is tapered from the first end edge 10a toward the second end edge 10b to prevent the layered both lateral regions 14 from increasing in thickness. The first, second and third regions 5, 6, 7 layered one upon another in the thickness direction Z are heat-sealed along the first and second side edges 2b, 2c overlapped to each other to form seal lines 9a, 9b so as to leave a take-out slot (the second end edge 2e). The take-out slot is sealed by pressure-sensitive adhesives distributed to the vicinity of an opening peripheral edge thereof and to the free portion 8b of the finger-grip tab 8. When it is desired to take out the male urine absorption pad 10 from the individually packaging envelope 2, the procedure to form the package 1 may be followed backward from the state illustrated in Fig. 8(e) toward the state illustrated in Fig. 8(a). Specifically, the finger-grip tab 8 is unsealed together with the opening peripheral edge of the take-out slot and the seal lines 9a, 9b are broken so that the third region 7 may be separated from the second surface 4 of the first region 5; the individually packaging envelope 2 is unfolded along the first fold lines 17a, 17b so as to spread out the envelope 2; then the pad main body 20 is unfolded along the second fold lines 18a, 18b so as to spread out the pad main body 20; and finally the separators 45 are peeled off from the associated attachment regions 40 and the male urine absorption pad 10 is taken out from the package 1. Referring to Fig. 8(f), the individually packaging envelope 2 and the separators 45 are bonded together by the adhesive regions 47 and, for this reason, it is possible to peel off the separators 45 together with the packaging sheet 2A from the pad main body 20 when the male urine absorption pad 10 is taken out from the individually packaging envelope 2.

Referring again to Fig. 3, in the layered state as illustrated, the central region 15 of the pad main body 20 is folded in three along the second fold lines 18 and then both the lateral regions 14 are folded together with the individually packaging envelope 2 along the first fold lines 17. Consequently, the package is compressed in the thickness direction Z and the regions folded along the second fold lines 18 likely leaves the folding propensity. For this reason, in the pad main body 20 spread out from the packaged state, the folding propensity left by the second fold lines 18 cause the first and second end regions 11, 12 to have postures, respectively, curved toward the skin-facing surface side of the intermediate region 13.

Referring again to Fig. 2, the first fold lines 17 is used after the pad main body 20 is folded in three and, for this reason, the regions folded along the first fold lines 17 are not so compressed as the regions folded along the second fold lines 18 in which the folding propensity are unavoidable. For this reason, none of the folding propensity due to the first fold lines 17 is left on the male urine absorption pad 10 spread out from the packaged state and none of the fold lines extending in the direction of the longitudinal center line P is formed between both the lateral regions 14 and the central region 15 which may deform these regions 14, 15. Particularly according to the present embodiment, the liquid-absorbent core 23 having a compressive elasticity is thinner in both the lateral regions 36 than in the central region 15 and there is no possibility that both the lateral regions 14 of the male urine absorption pad 10 may be entirely compressed as the central region 15 of the male urine absorption pad 10 is. For this reason, a folding propensity is scarcely left in both the lateral regions 14.

Assuming that the relatively thin both lateral regions 14 are overlapped on themselves, the regions folded along the first fold lines 17 is at a substantially the same level as the side edges 10c, 10d of both the lateral regions 14 and, in consequence, these folded regions is folded at a sufficiently acute angle to have a folding propensity. According to the present embodiment, however, both the lateral regions 14 partially overlap with the central region 15 of the liquid-absorbent core 23 having a thickness larger than in both the lateral regions 14 so that the side edges 10c, 10d of the respective lateral regions 14 may be sufficiently distanced from the regions folded along the first fold lines 17 in the thickness direction Z to ensure that these folded regions are gently curved and the unpacked and spreaded out pad main body 20 is substantially free from the folding propensity.

Referring to Figs. 9 and 10, in the male urine absorption pad 10 put on the wearer's body, the wearer's penis 80 is put in contact with the intermediate region 13 of the pad main body 20. The pad main body 20 has the first and second end regions 11, 12 curved toward the skin-facing surface side of the intermediate region 13 (i.e., toward the body side) under the effect of a folding propensity of the second fold lines 18a, 18b, making it easy for the intermediate region 13 to be deformed outward convexly along the wearer's body shape. The intermediate region 13 deformed outward convexly in a cup-like shape in this manner provides a good fit compared with when the intermediate region 13 is put in a planar contact with the penis 80, and, in addition, a gap causing a leakage of the body exudates may be left between the penis 80 and the intermediate region 13. Furthermore, both the lateral regions 14 of the first end region 11 are dimensioned to be wider than both the lateral regions 14 of the second end region 12 and both the lateral regions 14 of the intermediate region 11 the second end region 12 so that both the lateral regions 14 of the first end region 11 may be gently curved toward the wearer's body by the first fold lines 17a, 17b to form the cup-like shape convexed outward from both the lateral regions 14 toward the central region 15.

Referring to Fig. 11 (a) exemplarily illustrating a procedure to make the package, if both the lateral regions 14 are folded along the first fold lines 17a, 17b before the pad main body 20 is folded in three along the second fold lines 18a, 18b (one of the conventional procedure), the regions folded along the first fold lines 17a, 17b are so tightly compressed to have a folding propensity. In such an instance, the folded regions are angulated under the effect of the folding propensity of the first fold lines 17a, 17b, making it impossible to deform the pad main body 20 along the penis 80 protruding from the wearer's abdominal area, and a gap 60 causing urine-leakage may be left between the wearer's body and the pad main body 20.

Referring to Fig. 11 (b) illustrating the procedure according to the present invention to make the package 1, if both the lateral regions 14 are folded along the first fold lines 17a, 17b after the pad main body 20 are folded in three along the second fold lines 18a, 18b, the regions folded along the first fold lines 17a, 17b are free from any folding propensities and the male urine absorption pad 10 is deformed along the wearer's body shape. Consequently, no gaps causing leakage of body exudates are left between the wearer's body and the pad main body 20.

### <First Alternative>

Referring to Fig. 12(a), in the package 1 according to the present alternative, the adhesive regions 47 extend in the longitudinal direction (Y-direction) of the pad main body 20, overlap with the attachment regions 40 so as to be located on the side of the end edges 40a of the attachment regions 40 located on the side of the first end edge 10a of the pad main body 20, and overlap with at least one of both side edges 40c, 40d of the respective attachment regions 40. Each of the adhesive regions 47 is located across at least one of both the side edges 40c, 40d of the adhesive region 47 and, in consequence, it is possible to peel off the separators 45 arranged in the central attachment region 41 and the lateral attachment regions 42 smoothly irrespective of the direction in which the packaging sheet 2a is peeled off, i.e., whether peeling-off starts from the first end edge 2d or from the second end edge 2e.

### <Second Alternative>

Referring to Fig. 12(b), in the package 1 according to the present alternative, each of the adhesive regions 47 overlaps not only with the end edge 40a of the associated attachment region 40 but also with at least one of the side edges 40c, 40d of the associated attachment region 40. Such arrangement of the adhesive regions 47 makes it possible to peel off the separators 45 arranged in the attachment region 41 and the lateral attachment regions 42 not only when peeling-off of the packaging sheet 2A is started from the first side edge 2b of the packaging sheet 2A but also when peeling-off is started from any one of the first and second end edges 2d, 2e of the packaging sheet 2A.

### <Second Embodiment>

Referring to Fig. 13, according to the second embodiment of the present invention, the central attachment region 41 and the lateral attachment regions 42 are located at levels differing from each other in the direction of the longitudinal center line P so that the respective end edges 40a thereof located on the side of the respective first end edges 10a may be out of alignment in the direction of the lateral center line Q. Specifically, the end edge 40a of one of the lateral attachment regions 42 is located closer, compared with the respective end edges 40a of the other lateral attachment region 42 and the central attachment region 41, to the first end edge 10a, and the end edge 40a of the central attachment region 41 is located at a level between the levels of the respective end edges 40a of both the lateral attachment regions 42. By locating the respective end edges 40a of the attachment regions 40 at the levels differing from each other in the direction of the longitudinal center line P, it is possible to distribute a peel force acting on the central attachment region 41 and the lateral attachment regions 42 and thereby to peel off the packaging sheet 2A and the separators smoothly. In this regard, it is preferable that at least one of the end edges 40a of the central attachment region 41 and both the lateral attachment regions 42 is located closer, compared with the other end edges 40a, to the first end edge 10a and, for example, it is also possible to locate the end edge 40a of the central attachment region 41 closer, compared with the respective end edges 40a of both the lateral attachment regions 42, to the first end edge 10a and, for example, it is also possible to locate the end edge 40a of the central attachment region 41 closer, compared with the respective end edges 40a of both the lateral attachment regions 42, to the first end edge 10a.

### <Third Embodiment>

Referring to Fig. 14, the package 1 of the male urine absorption pad 10 according to the third embodiment of the present invention includes a pair of barrier-flap sheet (barrier-sheet) 50 and first and second cover sheets 61, 62 located in the first end region 11 and the second end region 12, respectively, to cover the pad main body 20. The pad main body 20, the barrier-flap sheets 50 and the first and second cover sheets 61, 62 are layered one on another and fixed in the layered state by a sealing line 19 such as thermal bonding line.

Referring to Fig. 14 and Fig. 15, each of the barrier-flap sheets 50 has a shape tapered from the first end edge 10a toward the second end edge 10b and formed of, for example, hardly-liquid-permeable or liquid-impermeable fibrous nonwoven fabrics. Each of the barrier-flap sheets 50 has a proximal edge portion 51 fixed together with the other sheet members along the sealing line 19, both end portions 52 fixed to the topsheet 21, for example, by hot melt adhesives in the first and second end regions 11, 12 and a sleeve-like distal edge portion 53 formed by folding back inward an inner side edge of the barrier-flap sheet 50 extending in the longitudinal direction between both the fixed end portions 52. Linear flap-elastic element 54 extending in the longitudinal direction is fixed to the distal edge portion 53 of the barrier-flap sheet 50. In the male urine absorption pad 10 put on the wearer's body, the distal edge portions 53 uprise off from the skin-facing surface side toward the wear's body side under a contractile effect to form a pair of barrier-cuff (3D-cuff) to prevent the urine from leaking sideways.

The first and second cover sheets 61, 62 respectively have both lateral portions 61a, 62a fixed to the barrier-flap sheets 50 on the side of the first end edge 10a and on the side of the second end edge 10b, respectively, and covering portions 61b, 61b adapted to cover the central region 15 of the pad main body 20 between both the fixed lateral portions 61a, 62a. As illustrated in Figs. 15 through 17, uprise of the barrier-flap sheets 50 causes the respective covering portions 61b, 62b of the first and second cover sheets 61, 62 to be spaced from the topsheet 21 lying in the central region 15 of the pad main body 20 so as to form first and second pockets 64, 65 between the covering portions 61b, 62b and the topsheet 21, respectively.

Referring to Fig. 16, the central region 35 of the absorbent core 23 has a high-absorption sub-region 85 on the side of the second end region 12, which is thicker than a remaining absorption sub-region 84 and has a liquid-absorption capacity correspondingly higher than that of the remaining absorption sub-region 84. An opening of the second pocket 65 formed by the second cover sheet 62 is located in this high-absorption sub-region 85. In this regard, it is possible within the scope of the present invention to locate the opening of the second pocket 65 in a boundary region 85a between the high-absorption sub-region 85 and the remaining absorption sub-region 84. The second fold line 18a is preferably located in a region wherein the mass per unit area of the liquid-absorbent core 23 undergoes a change, specifically, in the boundary region 85a between the high-absorption sub-region 85 and the other absorption sub-region 84 or in the absorption sub-region 84 in the vicinity of the boundary region 85a, in any instance, on the side closer, compared with flexion-inducing grooves 30 to be described hereinafter, to the second end edge 10b. Particularly for the reason that the second fold line 18a is arranged in the boundary region 85a, a stiffness change of the male urine absorption pad 10 occurring in the boundary region 85a and the folding propensity of the second fold line 18a make it easy to curve the second pocket 65 as a whole along the crotch region of the wearer's body, thereby providing a good fit further. In addition, with the arrangement such that the opening of the second pocket 65 is located in the high-absorption sub-region 85, i.e., the opening of the second pocket 65 is located closer, compared with the second fold line 18a, to the second end edge 10b, it is possible to prevent the folding propensity of the second folding line 18a from being left on the second cover sheet 62 and thereby to prevent the desired opening formation of the second pocket 65 from being restricted. Furthermore, as both the lateral portions of the second pocket 65 are wedged and flexed between the wearer's thighs, the covering portion 62b of the second cover sheet 62 is further convexed toward the wearer's body so as to widen the opening of the second pocket 65, reliably making it possible to guide the urine flowing down along the surface of the topsheet 21 into the second pocket 65. A mass per unit area of the liquid-absorbent core 23 in the high-absorption sub-region 85 is preferably in a range of 250 g/m² to 600 g/m². If the mass per unit area exceeds 600 g/m², the absorption pad 10 may become too bulky and cause a feeling of discomfort when the pad 10 is put on the wearer's body.

Referring again to Fig. 14, the flexion-inducing grooves 30 are arranged in the intermediate region 13 of the pad main body 20 in the package 1. The flexion-inducing grooves 30 are concave lines compressed from the topsheet 21 toward the liquid-absorbent core 23 and include a first groove 31 obliquely intersecting with the longitudinal center line P, a second groove 32 extending in a direction intersecting with the first groove 31, a third groove 33 located closer, compared with the first and second grooves 31, 32, to the second end edge 20b and a pair of fourth grooves 34 obliquely extending from the longitudinal center line P outward in the width direction of the pad main body 20. According to the present embodiment, the first and second grooves 31, 32 are discontinuously formed, more specifically, none of groove segments are formed in the crossover region M of the first and second grooves 31, 32.

Referring to Fig. 17, the intermediate region 13 of the male urine absorption pad 10 is convexly deformed outward (i.e., toward the side of the garment) by the flexion-inducing grooves 30 and a cup-shaped space adapted to receive the penis 80 in a wrapping up manner is formed in this convexly deformed region.

The first and second fold lines 17, 18 are arranged in regions not overlapping with the flexion-inducing grooves 30, specifically, arranged in a central sub-region 15 of the intermediate region 13 surrounded by the first and second fold lines 17, 18. Considering that the stiffness of the male urine absorption pad is unavoidably increases in the region including the flexion-inducing grooves 30, the first and second fold lines 17, 18 may be arranged in the portions not overlapping with such region to make easy to fold back the male urine absorption pad 10. In addition, the arrangement such that the flexion-inducing grooves 30 are formed not to overlap with the first and second fold lines 17, 18 make it possible for the male urine absorption pad 10 to form a stabilized cup-shape along the flexion-inducing grooves 30.

The disclosure relating to the present invention described above may be arranged at least as follows:
A package 1 includes a male urine absorption pad 10 having a skin-facing surface and a non-skin-facing surface opposite to the former and an individually packaging envelope 2 to package the male urine absorption pad 10. The individually packaging envelope 2 includes a packaging sheet 2 having a longitudinal direction X and a width direction X intersecting with the longitudinal direction X. The packaging sheet 2 has first and second end edges 2d, 2e spaced from and facing each other in the longitudinal direction X, a pair of first fold lines 17a, 17b extending in the width direction Y, a first region 5 defined between the first end edge 2d and one of the first fold lines 17a, 17a, a second region 6 defined between the pair of the first fold lines 17a, 17a and the third regions 7 defined between the second end edge 2e and the other first fold line 17b. The male urine absorption pad 10 has a pad main body 20 having liquid-absorbency. The pad main body 20 has first and second end edges 10a, 10b, both side edges 10c, 10d extending in the width direction Y, at least one second fold line 18 extending in the longitudinal direction X, both lateral regions 14 defined between both the side edges 10c, 10d and the pair of first fold lines 17a, 17b, a central region 15 defined between both the lateral regions 14, a first layer 16A fixed to the individual packaging envelope 2 and a second layer 16B flexed by the second fold line 18 and lapped on the first layer. After the second layer has been lapped on the first layer by the second fold line, the first, second and third regions 5, 6, 7 of the packaging sheet 2A are layered one on another, and whereby both the lateral regions 14 are folded back onto the central region 15.

The present invention disclosed in the paragraph {0051} may include embodiments at least as described below and these embodiments may be taken in isolation or in combination.
(1) The first layer 16A has a length dimension in a width direction (X-direction) of the pad main body 20 larger than that of the second layer 16B and both lateral regions 14 of the first layer 16A facing each other in the width direction are located outside both side edges 10c, 10d of the second layer 16B in the width direction.
(2) The second layer 16B is defined by a plurality of layers folded back onto each other.
(3) The pad main body 20 includes a liquid-permeable topsheet 21, a liquid-impermeable backsheet 22 and a liquid-absorbent core 23 sandwiched between the topsheet 21 and the backsheet 22 and wherein the first and second fold lines 17, 18 are located so as to overlap with the liquid-absorbent core 23.
(4) The liquid-absorbent core 23 has a central region 35 and both lateral regions 36 having a thickness dimension smaller than that of the central region 35 and the first fold lines 17 are located closer, compared with a boundary region between the central region 35 of the liquid-absorbent core 23 and both the lateral regions 36 of the liquid-absorbent core 23, to both the lateral regions 36 of the liquid-absorbent core 23.
(5) The central region 35 of the liquid-absorbent core 23 includes, on the side of the second end region 12, a high-absorption sub-region 85 having a mass per unit area larger than that of a remaining absorption sub-region 84; and one of the second fold lines is located in a boundary region between the high-absorption sub-region 85 and the remaining absorption sub-region 84.
(6) The male urine absorption pad 10 further includes a plurality of adhesive attachment regions 40 arranged on the non-skin-facing surface at given intervals in the width direction X of the pad main body 20 and separators 45 covering the attachment regions 40 and fixed to the packaging sheet 2A through the intermediary of adhesive portions 47.
(7) The adhesive portions 47 are arranged so as to overlap with respective end edges of the attachment regions 40 on the side of the first end edge 10a of the pad main body 20.
(8) The adhesive portions 47 are arranged so as to overlap with at least one of both side edges 40c, 40d of the respective attachment regions 40 facing each other in a width direction of the pad main body 20.
(9) The attachment regions 40 include a central attachment region 41 located in the central region 15 of the pad main body 20 and a pair of lateral attachment regions 42 arranged on both sides of the central attachment region 41 in such a manner that at least one of the end edges 40a of the central attachment region 41 and the lateral attachment regions 42 is out of alignment with the remaining end edges attachment regions in the longitudinal direction X.
(10) The pad main body 20 includes flexion-inducing grooves 30 arranged in the central region 15 of the pad main body 20 and being concave from the skin-facing surface toward the non-skin-facing surface and the first and second fold lines 17, 18 are located not so as to overlap with the flexion-inducing grooves.
(11) The pad main body 20 has a pair of the second fold lines 18a, 18b, an intermediate region 13 defined between these two second fold lines 18a, 18b, a first end region 11 defined between the first end edge 10a of the pad main body 20 and one of the second fold lines 18b and a second end edge 12 defined between the second end edge 10b of the pad main body 20 and the other of the second fold lines 18a; and the male urine absorption pad includes first and second cover sheets 61, 62 located in the first and second end regions 11, 12, respectively, and a pair of barrier-flap sheets 50 located both the lateral regions 14 of the pad main body 20.

### {Reference Signs List}

- 1: package of male urine absorption pad
- 2: individually packaging envelope
- 2A: packaging sheet
- 10: male urine absorption pad
- 10a: first end edge
- 10b: second end edge
- 11: first end region
- 12: second end region
- 13: intermediate region
- 14: both lateral regions
- 15: central region
- 16A: first layer
- 16B: second layer
- 17: first fold lines
- 18: second fold lines
- 20: pad main body
- 21: topsheet
- 22: backsheet
- 23: liquid-absorbent core
- 30: flexion-inducing grooves
- 40: attachment regions
- 41: central attachment region
- 42: lateral attachment regions
- 40a: end edges
- 45: separators
- 47: adhesive regions
- 50: barrier-flap sheets
- 61: first cover sheet
- 62: second cover sheet

## Claims

1. A package (1) including a male urine absorption pad (10) having a skin-facing surface and a non-skin-facing surface opposite to the former and an individually packaging envelope (2) to package the male urine absorption pad (10), wherein:
the individually packaging envelop (2) includes a packaging sheet (2A) having a longitudinal direction (X) and a width direction (Y) intersecting with the longitudinal direction (X);
the packaging sheet (2A) has first and second end edges (2d, 2e) spaced from and facing each other in the longitudinal direction (X), a pair of first fold lines (17a, 17b) extending in the width direction (Y), a first region (5) defined between the first end edge (2d) and one of the first fold lines (17a), a second region (6) defined between the pair of the first fold lines (17a, 17b) and the third regions (7) defined between the second end edge (2e) and the other first fold line (17b):
the male urine absorption pad (10) has a pad main body (20) having liquid-absorbency;
the pad main body (20) has first and second end edges (10a, 10b) extending in the longitudinal direction (X), both side edges (10c, 10d) extending in the width direction (Y), at least one second fold line (18a, 18b) extending in the longitudinal direction (X), both lateral regions (14) defined between both the side edges (10c, 10d) and the pair of first fold lines (17a, 17b), a central region (15) defined between both the lateral regions (14), a first layer (16A) fixed to the individual packaging envelope (2) and a second layer (16B) flexed by the second fold line (18a, 18b) and lapped on the first layer (16A); and
after the second layer (16B) has been layered on the first layer (16A) by the second fold line (18a, 18b), the first, second and third regions (4, 5, 6) of the packaging sheet (2) are layered one on another, and whereby both the lateral regions (14) are folded back onto the central region (15);
the pad main (20) body includes a liquid-permeable topsheet (21), a liquid-impermeable backsheet (22) and a liquid-absorbent core (23) interposed between the topsheet (21) and the backsheet (22) and wherein the first and second fold lines (17a, 17b, 18a, 18b) are located so as to overlap with the liquid-absorbent core (23); and
the liquid-absorbent core (23) has a central region (35) and both lateral regions (36) having a thickness dimension smaller than that of the central region (35) and the first fold lines (17a, 17b) are located closer, compared with a boundary region between the central region (35) of the liquid-absorbent core (23) and both the lateral regions (36) of the liquid-absorbent core (23), to both the lateral regions (36) of the liquid-absorbent core (23).

2. The package (1) according to Claim 1 wherein the first layer (16A) has a length dimension in a width direction (Y) of the pad main body (20) larger than that of the second layer (16B) and both lateral regions of the first layer (16A) facing each other in the width direction (Y) are located outside both side edges of the second layer (16B) in the width direction (Y).

3. The package (1) according to Claim 1 or 2 wherein the second layer (16B) is defined by a plurality of layers folded back onto each other.

4. The package according to Claim 1 , wherein the central region (35) of the liquid-absorbent core (23) includes, on the side of the second end edge (10b), a high-absorption sub-region (85) having a mass per unit area larger than that of a remaining absorption sub-region (84); and
one of the second fold lines (18a, 18b) is located in a boundary region (85a) between the high-absorption sub-region (85) and the remaining absorption sub-region (84).

5. The package (1) according to any one of Claims 1 through 4, wherein the male urine absorption pad (10) further includes a plurality of adhesive attachment regions (40) arranged on the non-skin-facing surface at given intervals in the width direction (Y) of the pad main body (20) and separators (45) covering the attachment regions (40) and fixed to the packaging sheet (2A) through the intermediary of adhesive portions (47).

6. The package (1) according to Claim 5, wherein the adhesive portions (47) are arranged so as to overlap with respective end edges (40a) of the attachment regions (40) on the side of the first end edge (10a) of the pad main body (20).

7. The package (1) according to Claim 5 or 6, wherein the adhesive portions (47) are arranged so as to overlap with at least one of both side edges (40c, 40d) of the respective attachment regions (40) facing each other in a width direction of the pad main body (20).

8. The package according to any one of Claims 5 through 7, wherein the attachment regions (40) include a central attachment region (41) located in the central region (15) of the pad main body (20) and a pair of lateral attachment regions (42) arranged on both sides of the central attachment region (41) in such a manner that at least one of the end edges (40a) of the central attachment region (41) and the lateral attachment regions (42) is out of alignment with the remaining end edges attachment regions in the longitudinal direction (X).

9. The package (1) according to any one of Claims 1 through 8, wherein the pad main body (20) includes flexion-inducing grooves (30) arranged in the central region (15) of the pad main body (20) and being concave from the skin-facing surface toward the non-skin-facing surface and the first and second fold lines (17a, 17b, 18a, 18b) are located not so as to overlap with the flexion-inducing grooves (30).

10. The package (1) according to any one of Claims 1 through 9, wherein the pad main body (20) has a pair of the second fold lines (17a, 17b, 18a, 18b), an intermediate region (13) defined between these two second fold lines (18a, 18b), a first end region (11) defined between the first end edge (10a) of the pad main body (20) and one of the second fold lines (18a, 18b) and a second end region (12) defined between the second end edge (10b) of the pad main body (20) and the other of the second fold lines (18a, 18b); and the male urine absorption pad (10) includes first and second cover sheets (61, 62) located in the first and second end regions (11, 12), respectively, and a pair of barrier-cuff sheets (50) located both the lateral regions (14) of the pad main body (20).

## Patentansprüche

1. Eine Verpackung (1), die ein Männerurinabsorptionskissen (10) mit einer Haut-zugewandten Oberfläche und einer nicht-Haut-zugewandten Oberfläche gegenüber der erstgenannten und einer individuellen Verpackungshülle (2), um das Männerurinabsorptionskissen (10) zu verpacken, umfasst, wobei:
die individuelle Verpackungshülle (2) eine Verpackungslage (2A) mit einer longitudinalen Richtung (X) und einer Breitenrichtung (Y), die sich mit der longitudinalen Richtung (X) schneidet, umfasst;
die Verpackungslage (2A) erste und zweite Kanten (2d, 2e), die voneinander beabstandet und in der longitudinalen Richtung (X) zueinander gerichtet sind, ein Paar von ersten Faltlinien (17a, 17b), die sich in der Breitenrichtung (Y) erstrecken, einen ersten Bereich (6), der zwischen der ersten Endkante (2d) und einer der ersten Faltlinien (17a) bestimmt ist, einen zweiten Bereich (6), der zwischen dem Paar von ersten Faltlinien (17a, 17b) bestimmt ist, und den dritten Bereichen (7), der zwischen der zweiten Endkante (2e) und der anderen ersten Faltlinie (17b) bestimmt ist, aufweist;
das Männerurinabsorptionskissen (10) einen Kissenhauptkörper (20) mit Flüssigkeits-Absorptionsfähigkeit aufweist;
der Kissenhauptkörper (20) erste und zweite Endkanten (10a, 10b), die sich in der longitudinalen Richtung (X) erstrecken, beide Seitenkanten (10c, 10d), die sich in Breitenrichtung (Y) erstrecken, wenigstens eine zweite Faltlinie (18a, 18b), die sich in der longitudinalen Richtung (X) erstreckt, beide Lateralbereiche (14), die zwischen beiden der Seitenkanten (10c, 10d) und dem Paar von ersten Faltlinien (17a, 17b) bestimmt sind, einen Zentralbereich (15), der zwischen beiden der Lateralbereiche (14) bestimmt ist, eine erste Schicht (16A), die an der individuellen Verpackungshülle (2) befestigt ist, und eine zweite Schicht (16B), die durch die zweite Faltlinie (18a, 18b) geknickt ist und die erste Schicht (16A) überlappt, aufweist; und
nachdem die zweite Schicht (16B) durch die zweite Faltlinie (18a, 18b) auf die erste Schicht (16A) geschichtet wurde, die ersten, zweiten und dritten Bereiche (4, 5, 6) der Verpackungslage (2) aufeinander geschichtet werden, und wodurch beide der Lateralbereiche (14) nach hinten auf den Zentralbereich (15) gefaltet werden;
der Kissenhauptkörper eine flüssigkeitsdurchlässige Oberlage (21), eine flüssigkeitsundurchlässige Unterlage (22) und einen flüssigkeitsabsorbierenden Kern (23), der zwischen der Oberlage (21) und der Unterlage (22) eingefügt ist, umfasst und wobei die ersten und zweiten Faltlinien (17a, 17b, 18a, 18b) so angeordnet sind, dass sie mit dem flüssigkeitsabsorbierenden Kern überlappen (23); und
der flüssigkeitsabsorbierende Kern (23) einen Zentralbereich (35) und beide Lateralbereiche (36), mit einer Dickendimension kleiner als die des Zentralbereichs (35), aufweist und die ersten Faltlinien (17a, 17b), im Vergleich mit einem Grenzbereich zwischen dem Zentralbereich (35) des flüssigkeitsabsorbierenden Kerns (23) und den beiden Lateralbereichen (36) des flüssigkeitsabsorbierenden Kerns (23), näher an den beiden Lateralbereichen (36) des flüssigkeitsabsorbierenden Kerns (23) angeordnet sind.

2. Die Verpackung (1) gemäß Anspruch 1, wobei die erste Schicht (16A) eine Längendimension in einer Breitenrichtung (Y) des Kissenhauptkörpers (20) größer als die der zweiten Schicht (16B) aufweist und beide Lateralbereiche der ersten Schicht (16A), die einander in der Breitenrichtung (Y) zugewandt sind, außerhalb beider Seitenkanten der zweiten Schicht (16B) in der Breitenrichtung (Y) angeordnet sind.

3. Die Verpackung (1) gemäß Anspruch 1 oder 2, wobei die zweite Schicht (16B) durch eine Vielzahl von aufeinander zurückgefalteten Schichten bestimmt ist.

4. Die Verpackung (1) gemäß Anspruch 1, wobei der Zentralbereich (35) des flüssigkeitsabsorbierenden Kerns (23), auf der Seite der zweiten Endkante (10b), einen hoch-absorptions Unterbereich (85) mit einem Flächengewicht größer als das des übrigen absorptions Unterbereich (84) umfasst; und
eine der zweiten Faltlinien (18a, 18b) in einem Grenzbereich (85a) zwischen dem hoch-absorptions Unterberiech (85) und dem übrigen absorptions Unterbereich (84) angeordnet ist.

5. Die Verpackung (1) gemäß einem der Ansprüche 1 bis 4, wobei das Männerurinabsorptionskissen (10) ferner eine Vielzahl von klebenden Befestigungsbereichen (40), die auf der nicht-Haut-zugewandten Oberfläche in bestimmten Abständen in der Breitenrichtung (Y) des Kissenhauptkörpers (20) angeordnet sind, und Abscheider (45), die die Befestigungsbereiche (40) abdecken und durch die Zwischenschaltung der Klebebereiche (47) an der Verpackungslage (2A) befestigt sind, umfasst.

6. Die Verpackung (1) gemäß Anspruch 5, wobei die Klebebereiche (47) so angeordnet sind, dass sie mit entsprechenden Endkanten (40a) der Befestigungsbereiche (40) auf der Seite der ersten Endkante (10a) des Kissenhauptkörpers (20) überlappen.

7. Die Verpackung (1) gemäß Anspruch 5 oder 6, wobei die Klebebereiche (47) so angeordnet sind, dass sie mit wenigstens einer der beiden Seitenkanten (40c, 40d) der entsprechenden Befestigungsbereiche (40) in einer Breitenrichtung des Kissenhauptkörpers (20) zueinander gerichtet überlappen.

8. Die Verpackung (1) gemäß einem der Ansprüche 5 bis 7, wobei die Befestigungsbereiche (40) einen Zentralbefestigungsbereich (41), der in dem Zentralbereich (15) des Kissenhauptkörpers (20) angeordnet ist, und ein Paar von Lateralbefestigungsbereiche (42), die auf beiden Seiten des Zentralbefestigungsbereichs (41) derart angeordnet sind, dass wenigstens eine der Endkanten (40a) des Zentralbefestigungsbereichs-(41) und der Lateralbefestigungsbereiche (42) nichtfluchtend mit den übrigen Endkantenbefestigungsbereichen in der longitudinalen Richtung (X) ist, umfasst.

9. Die Verpackung (1) gemäß einem der Ansprüche 1 bis 8, wobei der Kissenhauptkörper (20) beugungseinleitende Nuten (30), die in dem Zentralbereich (15) des Kissenhauptkörpers (20) angeordnet sind und konkav von der Haut-zugewandte Oberfläche in Richtung der nicht-Haut-zugewandten Oberfläche sind, umfasst und die ersten und zweiten Faltlinien (17a, 17b, 18a, 18b) nicht so angeordnet sind, dass sie mit den beugungseinleitenden Nuten (30) überlappen.

10. Die Verpackung (1) gemäß einem der Ansprüche 1 bis 9, wobei der Kissenhauptkörper (20) ein paar von zweiten Faltlinien (17a, 17b, 18a, 18b), einen Zwischenbereich (13), der zwischen diesen beiden zweiten Faltlinien (18a, 18b) bestimmt ist, einen ersten Endbereich (11), der zwischen der ersten Endkante (10a) des Kissenhauptkörpers (20) und einer der zweiten Faltlinien (18a, 18b) bestimmt ist, und einen zweiten Endbereich (12), der zwischen der zweiten Endkante (10b) des Kissenhauptkörpers (20) und der anderen der zweiten Faltlinien (18a, 18b) bestimmt ist, aufweist; und
das Männerurinabsorptionskissen (10) erste und zweite Decklagen (61, 62), die, entsprechend, in den ersten und zweiten Endbereichen (11, 12) angeordnet sind, und ein Paar von Sperrbundlagen (50), die in beiden der Lateralbereiche (14) des Kissenhauptkörpers (20) angeordnet sind, umfasst.

## Revendications

1. Emballage (1) comprenant une garniture d'absorption d'urine (10) pour hommes ayant une surface faisant face à la peau et une surface ne faisant pas face à la peau en regard de la première surface et une enveloppe de conditionnement individuel (2) permettant d'emballer la garniture d'adsorption d'urine (10) pour hommes dans lequel ;
l'enveloppe de conditionnement individuel (2) comprend une feuille de conditionnement (2A) ayant une direction longitudinale (X) et une direction transversale (Y) croisant la direction longitudinale (X) ;
la feuille de conditionnement (2A) présente des premier et second bords d'extrémité (2d, 2e) espacés l'un de l'autre et se faisant face dans la direction longitudinale (X), une paire de premières lignes de pliage (17a, 17b) s'étendant dans la direction transversale (Y), une première région (5) définie entre le premier bord d'extrémité (2d) et l'une des premières lignes de pliage (17a), une deuxième région (6) définie entre la paire des premières lignes de pliage (17a, 17b) et les troisièmes régions (7) définies entre le second bord d'extrémité (2e) et l'autre première ligne de pliage (17b) ;
la garniture d'absorption d'urine (10) pour hommes comprend un corps principal (20) de garniture présentant une capacité d'absorption de liquides :
le corps principal (20) de garniture comprend des premier et second bords d'extrémité (10a, 10b) s'étendant dans la direction longitudinale (X), les deux bords latéraux (10c, 10d) s'étendant dans la direction transversale (Y), au moins une seconde ligne de pliage (18a, 18b) s'étendant dans la direction longitudinale (X), les deux régions latérales (14) définies entre les deux bords latéraux (10c, 10d) et la paire de premières lignes de pliage (17a, 17b), une région centrale (15) définie entre les deux régions latérales (14), une première couche (16A) fixée à l'enveloppe de conditionnement individuel (2) et une seconde couche (16B) pliée par la seconde ligne de pliage (18a, 18b) et recouvrant la première couche (16A) ; et
une fois que la seconde couche (16B) a été couchée sur la première couche (16A) par la seconde ligne de pliage (18a, 18b), les première, deuxième et troisième régions (4, 5, 6) de la feuille de conditionnement (2) sont stratifiées l'une sur l'autre, et moyennant quoi les deux régions latérales (14) sont repliées sur la région centrale (15) ;
le corps principal (20) de garniture comprend une feuille supérieure perméable aux liquides (21), une feuille inférieure imperméable aux liquides (22) et une partie centrale absorbant les liquides (23) intercalée entre la feuille supérieure (21) et la feuille inférieure (22) et dans lequel les premières et secondes lignes de pliage (17a, 17b, 18a, 18b) sont disposées de sorte à recouvrir la partie centrale absorbant les liquides (23) ; et
la partie centrale absorbant les liquides (23) présente une région centrale (35) et deux régions latérales (36) ayant une dimension dans le sens de l'épaisseur inférieure à celle de la région centrale (35) et les premières lignes de pliage (17a, 17b) sont situées plus près des deux régions latérales (36) de la partie centrale absorbant les liquides (23), par comparaison avec une région de bordure entre la région centrale (35) de la partie centrale absorbant les liquides (23) et les deux régions latérales (36) de la partie centrale absorbant les liquides (23).

2. Emballage (1) selon la revendication 1, dans lequel la première couche (16A) a une dimension de longueur dans une direction transversale (Y) du corps principal (20) de garniture supérieure à celle de la seconde couche (16B) et les deux régions latérales de la première couche (16A) se faisant face dans la direction transversale (Y) sont disposées à l'extérieur des deux bords latéraux de la seconde couche (16B) dans la direction transversale (Y).

3. Emballage (1) selon la revendication 1 ou 2, dans lequel la seconde couche (16B) est définie par une pluralité de couches repliées les unes sur les autres.

4. Emballage selon la revendication 1, dans lequel la région centrale (35) de la partie centrale absorbant les liquides (23) comprend, du côté du second bord d'extrémité (10b), une sous-région d'absorption élevée (85) ayant une masse surfacique supérieure à celle d'une sous-région d'absorption restante (84) ; et
l'une des secondes lignes de pliage (18a, 18b) est située dans une région de bordure (85a) entre la sous-région d'absorption élevée (85) et la sous-région d'absorption restante (84).

5. Emballage (1) selon l'une quelconque des revendications 1 à 4, dans lequel la garniture d'absorption d'urine (10) pour hommes comprend en outre une pluralité de régions de fixation adhésives (40) agencées sur la surface ne faisant pas face à la peau à des intervalles donnés dans la direction transversale (Y) du corps principal (20) de garniture et des séparateurs (45) recouvrant les régions de fixation (40) et fixés à la feuille de conditionnement (2A) par l'intermédiaire des parties adhésives (47).

6. Emballage (1) selon la revendication 5, dans lequel les parties adhésives (47) sont agencées de sorte à recouvrir les bords d'extrémité respectifs (40a) des régions de fixation (40) du côté du premier bord d'extrémité (10a) du corps principal (20) de garniture.

7. Emballage (1) selon la revendication 5 ou 6, dans lequel les parties adhésives (47) sont agencées de sorte à recouvrir au moins un des deux bords latéraux (40c, 40d) des régions de fixation respectives (40) se faisant face dans une direction transversale du corps principal (20) de garniture.

8. Emballage selon l'une quelconque des revendications 5 à 7, dans lequel les régions de fixation (40) comprennent une région de fixation centrale (41) située dans la région centrale (15) du corps principal (20) de garniture et une paire de régions de fixation latérales (42) agencées des deux côtés de la région de fixation centrale (41) de telle manière à ce qu'au moins un des bords d'extrémité (40a) de la région de fixation centrale (41) et des régions de fixation latérales (42) ne soit pas aligné avec les régions de fixation des bords d'extrémité restants dans la direction longitudinale (X).

9. Emballage (1) selon l'une quelconque des revendications 1 à 8, dans lequel le corps principal (20) de garniture comprend des rainures induisant une flexion (30) agencées dans la région centrale (15) du corps principal (20) de garniture et étant concaves, en allant de la surface faisant face à la peau vers la surface ne faisant pas face à la peau, et les premières et secondes lignes de pliage (17a, 17b, 18a, 18b) sont disposées de sorte à ne pas recouvrir les rainures induisant une flexion (30).

10. Emballage (1) selon l'une quelconque des revendications 1 à 9, dans lequel le corps principal (20) de garniture comprend une paire des secondes lignes de pliage (17a, 17b, 18a, 18b), une région intermédiaire (13) définie entre ces deux secondes lignes de pliage (18a, 18b), une première région d'extrémité (11) définie entre le premier bord d'extrémité (10a) du corps principal (20) de garniture et l'une des secondes lignes de pliage (18a, 18b) et une seconde région d'extrémité (12) définie entre le second bord d'extrémité (10b) du corps principal (20) de garniture et l'autre des secondes lignes de pliage (18a, 18b) ; et la garniture d'absorption d'urine (10) pour hommes comprend des première et seconde feuilles de couverture (61, 62) disposées dans les première et seconde régions d'extrémité (11, 12), respectivement, et une paire de feuilles revers formant barrière (50) disposée dans les deux régions latérales (14) du corps principal (20) de garniture.
